(19) Europäisches Patentamt
European Patent Office
Office européen des brevets

(11) Veröffentlichungsnummer: **0 396 014**
**A2**

(12) # EUROPÄISCHE PATENTANMELDUNG

(21) Anmeldenummer: **90107910.3**

(51) Int. Cl.5: **C07C 63/72, C07C 65/24**

(22) Anmeldetag: **26.04.90**

(30) Priorität: **29.04.89 DE 3914227**

(43) Veröffentlichungstag der Anmeldung: **07.11.90 Patentblatt 90/45**

(84) Benannte Vertragsstaaten: **AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Lerch, Ulrich, Dr.**
**Vorderwart 24**
**D-6238 Hofheim am Taunus(DE)**

(54) **Substituierte Biphenylcarbonsäuren, Verfahren zu ihrer Herstellung und neue Zwischenprodukte.**

(57) Neue Biphenylcarbonsäuren der Formel I

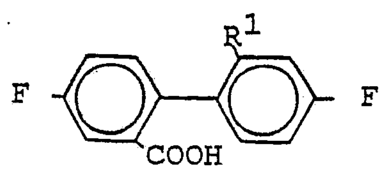

I

worin R1 die angegebenenen Bedeutungen hat und ein Verfahren zu ihrer Herstellung werden beschrieben.

EP 0 396 014 A2

**Substituierte Biphenylcarbonsäuren, Verfahren zu ihrer Herstellung und neue Zwischenprodukte**

Die Erfindung betrifft substituierte Biphenylcarbonsäuren der Formel I

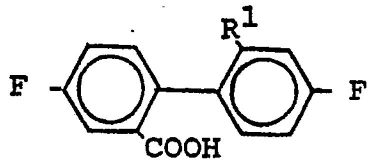

I

worin $R^1$ Wasserstoff oder Methoxy bedeutet, sowie Verfahren zu ihrer Herstellung.

Die substituierten Biphenylcarbonsäuren der Formel I lassen sich durch Cyclisierung in die entsprechenden 2,7-Difluor-fluorenone der Formel IX

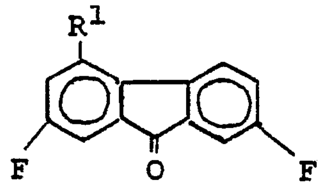

IX

überführen. Aus diesen lassen sich in einem einzigen Reaktionsschritt 2,7-Difluor-spiro[9H-fluoren-9,4′-imidazolidin]-2′,5′-dion (auch als AL 1576, HOE 843 und Imirestat bezeichnete), bzw. 2,7-Difluor-4-methoxy-spiro [9H-fluoren-9,4′-imidazolidin]-2,5′-dion herstellen, die als Arzneimittel zur Behandlung von Diabetes-Spätschäden verwendet werden können (vgl. US-Patentschrift 4.438.272).

Das aus der 4,4′-Difluorbiphenyl-2-carbonsäure leicht herzustellende 2,7-Difluorfluorenon läßt sich entsprechend dem Stand der Technik in einer 9-stufigen Synthese aus Fluoren herstellen, und zwar durch Nitrierung in 2-Stellung, Reduktion zum Amin, Diazotierung und Schiemann-Reaktion zum 2-Fluorfluorenon, anschließend Nitrierung in 7-Position und Wiederholung der genannten Reaktionssequenz zum 2,7-Difluorfluoren sowie abschließende Oxydation zum 2,7-Difluor-9-fluorenon IX mit Natriumdichromat (vgl. z.B. Chem. Ind. 1961, 179, J. Med. Chem. 8, 491 (1965), Isr. J. Chem. 1,1 (1963) und Chem. Ber. 112, 3490 (1979)).

Dieses Herstellungsverfahren ist vielstufig und die Gesamtausbeute liegt z.T. aufgrund von notwendigen Isomerentrennungen auf der Stufe der Nitroverbindungen bei 10%.

Ein in der US-patentschrift 4.438.272 beschriebenes Verfahren zur Herstellung von 2,7-Difluor-9-fluorenon geht von 2,7-Diaminofluoren aus, das durch Behandlung mit Fluorborsäure in das entsprechende Salz der Fluorborsäure überführt wird. Durch anschließende Diazotierung kommt man zum Bis-Diazoniumsalz, aus dem unter Erwärmen in Xylol das 2,7-Difluorfluoren freigesetzt wird. Die Aufoxydation zum 2,7-Difluor-9-fluorenon erfolgt mit $KMnO_4$ in Pyridin.

Das bei dieser Synthese als Ausgangsmaterial verwendete 2,7-Diaminofluoren ist allerdings durch Nitrierung von Fluoren zum 2,7-Dinitrofluoren und anschließende Reduktion nur schwierig und in unbefriedigenden Ausbeuten herstellbar, da bei der Nitrierung Isomerengemische entstehen, die aufgetrennt werden müssen und da das 2,7-Diaminofluoren sehr licht- und oxydationsempfindlich ist. Die Gesamtausbeuten basierend auf Fluoren liegen auch bei diesem Verfahren nur bei etwa 10 %.

Ein besonders ernstzunehmender Nachteil der bekannten Synthesen der Fluorenone der Formel IX liegt in der Tatsache, daß Amino- und Nitrofluorenderivate krebseregende Eigenschaften besitzen (vgl. J. Natl. Cancer Inst. 10, 1201 (1950), Cancer Res. 22, 1002 (1962), Brit. J. Cancer 4, 235 (1950), Brit. J. Exptl. Path. 25, 1 (1944) und Progressus Med. 3, 79 (1940)).

Die Herstellung des 2,7-Difluor-4-methoxy-9-fluorenons erfolgt über 4 bzw. 5 weitere Reaktionsschritte aus dem 2,7-Difluor-9-fluorenon.

Aufgabe der vorliegenden Erfindung ist es, ein wirtschaftliches und in großem Maßstab durchführbares Verfahren zur Herstellung von 2,7-Difluor-9-fluorenonen der Formel IX zur Verfügung zu stellen, bei dem die Nachteile, die bei der Herstellung gemäß dem Stand der Technik auftreten, vermieden werden. Das bedeutet, daß die Gesamtausbeute hoch ist, das Produkt in hoher Reinheit anfällt und daß keine bekanntermaßen cancerogene Zwischenprodukte auftreten.

Diese Aufgabe wird erfindunsggemäß dadurch gelöst, daß neue Biphenylcarbonsäuren der Formel I zur Verfügung gestellt werden, die auf einfache Weise durch Cyclisierung in die entsprechenden Fluorenone überführt werden, wobei diese in hohen Ausbeuten anfallen. Bei der Herstellung der erfindungsgemäßen Biphenylcarbonsäuren der Formel I sowie bei deren Weiterverarbeitung zu den Fluorenonen IX treten die geschilderten Nachteile nicht auf.

Die Erfindung betrifft daher die Biphenylcarbonsäuren der Formel I und ein Verfahren zu deren Herstellung.

Das Verfahren zur Herstellung von substituierten Biphenylcarbonsäuren der Formel I ist dadurch gekennzeichnet, daß man

a) eine 2-Methoxybenzoesäure der Formel II

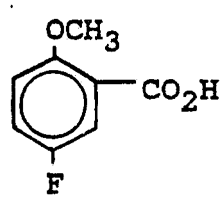

II

oder ein reaktives Derivat dieser Säure mit einem Ethanolamin der Formel III

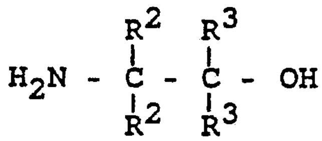

III

worin $R^2$ und $R^3$ Wasserstoff oder $(C_1\text{-}C_3)$-Alkyl, vorzugsweise Methyl, bedeuten, wobei jedoch $R^2$ und $R^3$ nicht gleichzeitig Alkyl bedeuten, zu einem Amid der allgemeinen Formel IV

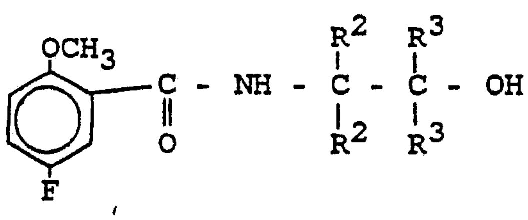

IV

worin $R^2$ und $R^3$ die zur Formel III angegebenen Bedeutungen haben, umsetzt, das Amid der Formel IV anschließend zu einem Dihydrooxazol der Formel V

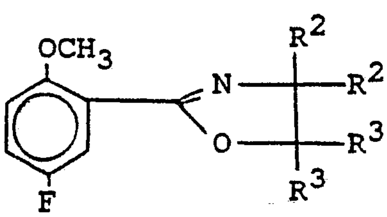

V

worin $R^2$ und $R^3$ die zur Formel III angegebenen Bedeutungen haben, cyclisiert, das Dihydrooxazol der Formel V anschließend mit einem metallorganischen Reagens der Formel VI

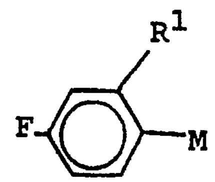

VI

worin $R^1$ die zur Formel I angegebene Bedeutung hat und M Lithium oder Zn-Hal oder vorzugsweise Mg-Hal und Hal Chlor, Brom oder Jod bedeuten, zu einem Biphenylderivat der allgemeinen Formel VII

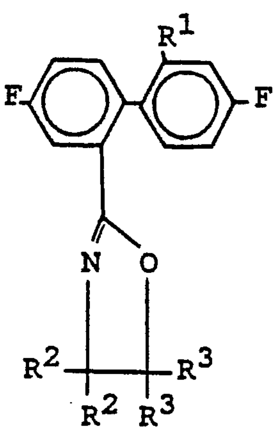

VII

worin $R^1$ die zur Formel I und $R^2$ und $R^3$ die zur Formel III angegebenen Bedeutungen haben, umsetzt, das erhaltene Biphenylderivat der Formel VII gegebenenfalls nach Überführung in die entsprechende alkylierte Verbindung der Formel VIII

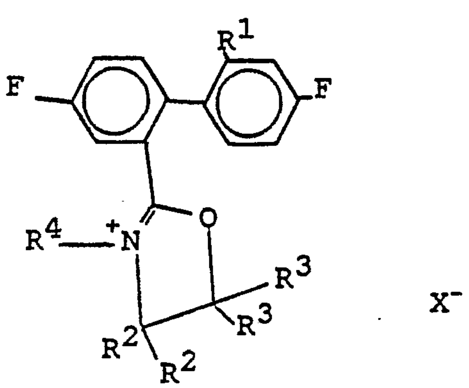

VIII

worin $R^1$ die zur Formel I und $R^2$ und $R^3$ die zur Formel III angegebenen Bedeutungen haben, $R^4$ $(C_1-C_3)$-Akyl, vorzugsweise Methyl, bedeutet und X- Chlorid, Bromid, Jodid, Methylsulfat oder Ethylsulfat bedeutet, zur Carbonsäure der Formel I

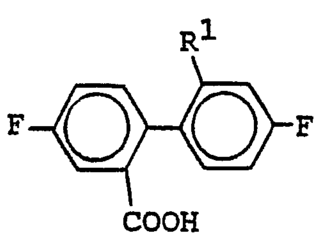

I

worin $R^1$ die zur Formel I angegebene Bedeutung hat, hydrolysiert.

Die Biphenylcarbonsäuren der Formel I können in an sich bekannter Weise anschließend direkt, über

das entsprechende Säurechlorid oder das Amid zum substituierten Fluorenon der Formel IX cyclisiert werden.

Das erfindungsgemäße Verfahren besitzt den Vorteil, daß es von entsprechend substituierten Ausgangsmaterialien ausgeht und in gezielter Weise zu reinen Biphenylcarbonsäuren der Formel I führt, in denen die Positionen der Substituenten eindeutig durch die Synthese festgelegt sind. Aufwendige und verlustreiche Isomerentrennungen sind nicht erforderlich und die Gesamtausbeute (etwa 65 %) liegt deutlich höher als bei den bekannten Verfahren (etwa 10 %). Nach dem erfindungsgemäßen Verfahren müssen z.B. zur Synthese von 4,4′-Difluorbiphenyl-2-carbonsäure ausgehend von 5-Fluor-2-methoxybenzoesäure nur zwei isolierte Zwischenstufen durchlaufen werden (Verbindungen V und VII).

Die 4,4′Difluorbiphenylcarbonsäuren der Formel I fallen nach dem erfindungsgemäßen Verfahren in sehr reiner Form an. Dies gilt auch für die daraus herstellbaren Fluorenone.

Besonders wichtig ist, daß die bekannten cancerogenen Nitro- und Aminofluorenderivate nicht durchlaufen werden.

Im einzelnen wird nach dem erfindungsgemäßen Verfahren die Herstellung des Benzamids IV aus der Benzoesäure II mit dem Aminoalkohol III in an sich bekannter Weise durchgeführt, z.B. läßt sich die Umsetzung mit einem ($C_1$-$C_4$)-Alkylester von II mit dem Aminoalkohol III durch Erhitzen der Komponenten in Anwesenheit oder Abwesenheit eines Lösungsmittels durchführen. Amide der Formel IV kann man auch durch Reaktion der Säure II mit einem Säurechlorid, wie Chlorameisensäureester oder Toluolsulfochlorid, in Gegenwart einer Base wie z.B. Triethylamin, in ein gemischtes Anhydrid überführen, das anschließend mit einem Amin der Formel III zu den Amiden IV reagiert. In einer bevorzugten Ausführungsform des erfindungsgemäßen Verfahrens wird die Säure II z.B. mit $PCl_5$ oder vorzugsweise Thionylchlorid in das entsprechende Säurechlorid überführt, das in fast quantitativer Ausbeute mit einem Amin III das entsprechende Amid IV liefert.

Die Cyclisierung von Amiden der Formel IV zu den Dihydrooxazolen V kann in an sich bekannter Weise mit wasserbindenden Mitteln, wie konzentrierter Schwefelsäure, Polyphosphorsäure oder Phosphorpentoxyd durchgeführt werden. Bevorzugt ist die Cyclisierung mit Thionylchlorid in einem inerten Lösungsmittel wie z.B. Diethylether, Methylenchlorid oder Toluol. Sie kann bei Temperaturen zwischen -10 und 50 °C, vorzugsweise bei 0 bis 30 °C durchgeführt werden. Aus dem zunächst entstehenden Hydrochlorid wird das freie Dihydrooxazolderivat V mit Basen wie z.B. Natronlauge hergestellt.

In einer besonders bevorzugten Ausführungsform des erfindungsgemäßen Verfahrens werden die Dihydrooxazole V aus der Benzoesäure II hergestellt, ohne die Amide der Formel IV zu isolieren.
Im einzelnen wird dabei die Benzoesäure II mit einem geringen überschuß an Thionylchlorid in Abwesenheit oder Anwesenheit eines inerten Lösungsmittels wie Methylenchlorid oder Toluol erhitzt und die Lösung des entstandenen Säurechlorids zu dem Aminoalkohol III zugetropft. Da der Aminoalkohol auch als Säurefänger fungiert, wird er in doppelt molarer Menge eingesetzt. Eine Alternative dazu ist die Verwendung von je einem Mol Aminoalkohol III und einem Mol einer inerten Base wie Triethylamin. Durch nachfolgende Zugabe der molaren Menge Thionylchlorid in die Reaktionsmischung erfolgt Ringschluß zum Dihydrooxazol V. Nach Alkalischstellen der Reaktionsmischung mit Natronlauge und Eindampfen der organischen Phase erhält man das Dihydrooxazol V in fast quantitativer Ausbeute. Das Produkt kann durch Destillation gereinigt werden.

Überraschenderweise verläuft die Umsetzung des Dihydrooxazols V mit einem metallorganischen Reagenz VI einheitlich ab unter Substitution der Methoxygruppe, wobei Biphenylderivate der Formel VII erhalten werden. Nennenswerte Nebenreaktionen durch nucleophilen Austausch z.B. eines Fluoratoms in den Verbindungen V oder VII treten nicht auf. Als Lösungsmittel für diese Umsetzung können Ether, wie Diethylether, Methyl-t-butylether oder Dibutylether, verwendet werden, bevorzugt ist Tetrahydrofuran. Mit Magnesium- oder Zinkorganylen erfolgt die Reaktion bei Temperaturen zwischen 0 °C und der Siedetemperatur des Lösungsmittels, mit 4-Fluorphenyllithium läßt man zwischen -60 °C und Raumtemperatur reagieren. Die metallorganischen Reagenzien VI werden zur Erzielung einer befriedigenden Ausbeute vorzugsweise im Überschuß eingesetzt, und zwar 1,2 bis 3,0 Mol, vorzugsweise 2-3 Mol, pro Mol Dihydrooxazol V. Die Reaktion kann in Gegenwart oder Abwesenheit einer katalytischen Menge von Palladiumverbindungen wie z.B. Pd $(PPh_3)_4$, erfolgen

Bevorzugt ist die Bildung der Biphenylderivate VII aus den Dihydrooxazolen V und dem Grignard-Reagens VI (M = Mg Br). Dabei kann das Grignard-Reagens zum Dihydrooxazol zugegeben werden oder das Dihydrooxazol V zum Grignard-Reagens.

In einer besonders bevorzugten Ausführungsform des erfindungsgemäßen Verfahrens wird z.B. bei der Herstellung von 4,4′-Difluorbiphenyl-2-carbonsäure das Magnesium mit der gesamten Menge an THF vorgelegt und Vitride® ($NaAlH_2$ $(OCH_2CH_2OCH_3)_2$ 70 %ig in Toluol zugegeben, bis die Wasserstoffentwicklung beendet ist. In das siedende THF läßt man anschließend das 4-Bromfluorbenzol laufen, wobei die

Grignardreaktion sofort anspringt. Nach beendeter Bildung des Grignard-Reagens wird das Dihydrooxazol V mit oder vorzugsweise ohne Lösungsmittel zugegeben. Nach kurzer Zeit (etwa 10 Minuten) ist die Reaktion beendet. Es wird mit Wasser oder gesättigter Ammoniumchlorid-Lösung hydrolysiert und mit Salzsäure auf pH 4-7 gestellt, um eine gute Phasentrennung zu erzielen. Das Produkt kann durch Kristallisation und/oder Destillation gereinigt werden.

Die Verseifung des Dihydro-1,3-oxazols VII zu der Biphenylcarbonsäure I erfolgt durch Hydrolyse mit wäßrigen Säuren, z.B. durch Erhitzen mit Salzsäure, Schwefelsäure oder Methansulfonsäure.

Bei einer bevorzugten Variante des erfindungsgemäßen Verfahrens werden Verbindungen der allgemeinen Formel VII mit Alkylierungsmitteln wie z.B. ($C_1$-$C_3$)-Alkylhalogeniden, Methansulfonsäure($C_1$-$C_3$)-alkylestern, Toluolsulfonsäure($C_1$-$C_3$) alkylestern oder Di($C_1$-$C_3$)alkylsulfaten zu Verbindungen der allgemeinen Formel VIII umgesetzt. Besonders bevorzugt als Alkylierungsmittel sind Dimethylsulfat, Methyljodid, Methylbromid und Methylchlorid in Lösungsmitteln wie Toluol, Aceton, 2-Butanon oder niedermolekularen Alkoholen. Die Alkylierungsreaktion kann in Gegenwart oder bevorzugt in Abwesenheit einer Base wie Natronlauge, Kaliumcarbonat oder Triethlyamin durchgeführt werden, ggf. auch in Gegenwart von Phasentransfer-Katalysatoren wie Tetrabutlyammoniumsulfat. Das entstehende Dihydro-1,3-oxazoliumsalz VIII wird anschließend zur Biphenylcarbonsäure I hydrolysiert. Dies kann durch Erhitzen mit wäßrigen Alkalihydroxydlösungen, wie Natronlauge oder Kalilauge, erfolgen, vorzugsweise im Sinn einer Eintropfreaktion, ohne das Salz VIII zu isolieren.

Der Ringschluß der Carbonsäure I zum Fluorenon IX erfolgt in an sich bekannter Weise z.B. mit wasserabspaltenden Mitteln wie z.B. in Polyphosphorsäure, bei Temperaturen zwischen etwa 80 - 120 °C.

Die Verbindungen der Formeln V, VII und VIII sind neu und stellen wertvolle Zwischenprodukte z.B. zur Herstellung von Biphenylcarbonsäuren der Formel I dar. Die Erfindung betrifft daher auch diese Zwischenprodukte und Verfahren zu ihrer Herstellung.

**Beispiel I**

**4,4′-Difluorbiphenyl-2-carbonsäure**

1) **5-Fluor-2-methoxybenzoesäure-2-(1-hydroxy-2-methyl) propylamid (IV)**

23,0 g (0,135 Mol) 5-Fluor-2-methoxybenzoesäure werden in 50 ml Methylenchlorid gelöst. Bei Raumtemperatur setzt man 17,6 ml (0,2 Mol) Thionylchlorid und 2 Tropfen DMF zu und erwärmt langsam zum Sieden. Nach beendeter Reaktion (Dünnschichtkontrolle nach Zusatz von Methanol zu einer Probe der Reaktionsmischung) wird i. Vak. eingedampft und die Thionylchloridreste werden nach zweimaliger Zugabe von weiteren Methylenchlorid i.Vak. entfernt. Der Rückstand (28,7 g) wird in 100 ml Methylenchlorid gelöst und die erhaltene Lösung bei 0-5 °C zu einer Lösung von 24,06 g (0,27 Mol) 2-Amino-2-methyl-1-propanol in 50 ml Methylenchlorid zugetropft. Man rührt 1 Std. ohne Eiskühlung nach und wäscht zweimal mit je 100 ml Wasser, danach mit 50 ml verd. HCl, trocknet über wasserfreiem Natriumsulfat und engt i. Vak. ein. Der krist. Rückstand (30,5 g, 93,5 % d. Th.) ist für die weiteren Umsetzungen genügend rein.
Fp. 97 - 98 °C (aus Isopropanol)
$^1$H-NMR: $\delta$ = 4,8 ppm (t, 1H), 3,95 ppm (s, 3H), 3,65 ppm (d,2H) 1,4 ppm (s, 6H)

2) **2-(5-Fluor-2-methoxyphenyl)-4,4-dimethyl-4,5-dihydro-1,3oxazol (V)**

a) Zu 14,4 ml (0,17 Mol) Thionylchlorid gibt man bei 20 -25 °C portionsweise 20 g (0,083 Mol) 5-Fluor-2-methoxybenzoesäure-2-(1-hydroxy-2-methyl)-propylamid, wobei zeitweise mit Eiswasser gekühlt werden muß. Eine Stunde wird bei Raumtemperatur nachgerührt, danach wird zweimal je 100 ml Äther zugegeben, gerührt und danach jeweils die Ätherphase abdekantiert. Der halbkristalline Rückstand wird in 100 ml Methylenchlorid aufgenommen. Die Lösung tropft man unter Eiskühlung bei etwa 15 °C zu 150 ml halbkonzentierter Natronlauge. Am Ende des Zutropfens muß der pH-Wert der wäßrigen Phase über 10 liegen. Die wäßrige Phase wird abgetrennt und nochmals mit Methylenchlorid extrahiert. Die vereinigten organischen Phasen werden mit gesättigter Natriumchlorid-Lösung gewaschen, über wasserfreiem Natriumsulfat getrocknet und i. Vak. eingedampft. Man erhält 18,7 g eines Öles, das allmählich kristallisiert. Nach Destillation schmilzt das Produkt bei 35 - 36 °C. $Sdp_{0,5}$ 98 °C

Ausbeute: Rohprodukt 93 - 100 %, nach Destillation 85 - 95 %.
$^1$H-HMR: $\delta$ = 6,6 - 7,6 ppm (m, 3H), 4,05 ppm (s, 2H) 3,8 ppm (s, 3H), 1,35 ppm (s, 6H)

b) (Eintopfreaktion)

230 g 5-Fluor-2-methoxybenzoesäure werden in 100 ml Methylenchlorid und 1 ml DMF gelöst. Dazu gibt man 108 ml Thionylchlorid. Man erhitzt unter Rückfluß bis die Säurechloridbildung abgeschlossen ist (etwa 1 Stunde). Zu der klaren Reaktionslösung läßt man anschließend bei 10 °C eine Lösung von 293,5 ml 2-Amino-2-methyl-1-propanol in 200 ml Methylenchlorid zulaufen. Nach beendeter Zugabe wird 30 Minuten bei Raumtemperatur nachgerührt, und danach werden 98 ml Thionylchlorid bei 20 °C zugetropft, Es wird 15 Minuten nachgerührt und es werden etwa 250 ml konz. Natronlauge zugetropft bis ein pH-Wert von >10 erreicht ist. Die organische Phase wird einmal mit verdünnter Natronlauge gewaschen, über Natriumsulfat getrocknet und i.Vak. destilliert. Ausbeute 276 g (92,9 %).

3) **2-(4,4'-Difluorbiphenyl-2-yl)-4,4-dimethyl-4,5-dihydro-1,3-oxazol VII**

1,18 kg Magnesiumspäne werden in 25 l THF vorgelegt und auf 50-60 °C erwärmt. Es wird Vitride-Lösung in Toluol (70 %ig) solange zugegeben, bis die Wasserstoffentwicklung beendet ist (etwa 150 ml). Man erhitzt die Mischung unter Rückfluß und gibt bei kräftigem Rühren 500 g 4-Bromfluorbenzol dazu. Die exotherme Reaktion springt fast augenblicklich an. Man läßt weitere 7,99 kg 4-Bromfluorbenzol so zulaufen, daß die Mischung immer am Rückfluß kocht. Nach beendeter Zugabe wird 1 Stunde weiter erhitzt. Danach werden 3,6 kg 2-(5-Fluor-2-methoxyphenyl)-4,4-dimethyl-4,5-dihydro-1,3-oxazol in 5 l trockenem THF bei Rückflußtemperatur zudosiert und 15 Minuten weiter erhitzt. Die Mischung wird auf 10 °C abgekühlt. Bei 10 - 20 °C werden 20 l gesättigte Ammoniumchloridlösung unter guter Kühlung zudosiert und mit etwa 3,5 l konz. Salzsäure wird der pH-Wert auf 6 eingestellt. Die ausgefallenen Salze werden mit Wasser gelöst und die Phasen getrennt. Die organische Phase wird mit 9 l gesättigter Natriumchloridlösung gewaschen, mit 200 g Aktivkohle verrührt, mit Natriumsulfat getrocknet und abgesaugt. Nach Verdampfen des Lösungsmittels und Trocknen i. Vak. erhält man 4,5 kg gelbes, kristallines Produkt, das für die weiteren Umsetzungen genügend rein ist (97 %ig nach GC-Analyse, Ausbeute 94,2 %). Es kann durch Kristallisieren aus Isopropanol oder Diisopropylether und Destillation der Mutterlauge am Dünnschichtverdampfer gereinigt werden. Sdp. 126 - 130 °C (0,1 mm)
Fp. 83 - 84 °C
$^1$H-NMR: $\delta$ = 6,4 - 7,5 ppm (m, 7H), 3,85 ppm (s, 2H), 1,28 ppm (s, 6H)

4. a) **2-(4,4'-Difluorbiphenyl-2-yl)-3,4,4-trimethyl-4,5-dihydro-1,3-oxazolium-jodid (VIII, $R^1$ = H, $R^2$, $R^4$ = $CH_3$, $R^3$ = H, X = $J^{(-)}$)**

8,6 g 2-(4,4'-Difluorbiphenyl-2-yl)-4,4-dimethyl-4,5-dihydro-1,3-oxazol werden in 60 ml Aceton und 30 ml Methyljodid 2 Tage unter Rückfluß gerührt. Man engt i. Vak. ein und kristallisiert den öligen Rückstand durch Zugabe von wenig Aceton. Nach Absaugen und Waschen mit wenig Aceton erhält man 5,8 g Kristalle mit Fp. 179 °C.
$^1$H-NMR: $\delta$ = 8,3 - 8,6 (m, 1H), 7,1 - 7,6 (m, 6H), 5,25 (s, 2H), 2,85 (s, 3H), 1,6 (s, 6H).

b) **2-(4,4'-Difluorbiphenyl-2-yl)-3,4,4-trimethyl-4,5-dihydro-1,3-oxazolium-methylsulfat (VIII, $R^1$ = H, $R^2$, $R^4$ = $CH_3$, $R^3$ = H, X = $SO_4^{2-}$)**

4,3 g des Dihydro-oxazols (aus 3.) werden in 30 ml Toluol und 1,7 l ml Dimethylsulfat zwei Stunden unter Rückfluß gerührt. Über Nacht kristallisiert das Salz aus. Man saugt ab, wäscht mit wenig Aceton und erhält 5,65 g (92, 6 %) Produkt. Es ist für die weiteren Umsetzungen genügend rein. Nach Umkristallisieren aus Isopropanol schmilzt es bei 167 - 169 °C.
$^1$H-NMR: $\delta$ = 8,05 - 8,35 (m, 1H), 7,0 - 7,6 (m, 6H), 5,05 (s, 2H), 3,7 (s, 3H), 2,75 (s, 3H), 1,5 (s, 6H).

**5. 4,4'-Difluorbiphenyl-2-carbonsäure (I)**

a) 7,18 g 2-(4,4'-Difluorbiphenyl-2-yl)-4,4'-dimethyl-4,5-dihydro-1,3-oxazol (Verb. VII) werden in 75 ml Dioxan und 80 ml halbkonzentrierter Salzsäure 3 Tage unter Rückfluß verseift. Das Lösungsmittel wird i. Vak. entfernt, der Rückstand wird in wenig halbkonzentrierter Natronlauge aufgenommen, die Lösung zweimal mit je 50 ml Ether gewaschen, mit Aktivkohle geklärt und mit verdünnter Salzsäure angesäuert. Man saugt ab und wäscht mit Wasser. Man erhält farblose Kristalle mit Fp. 117 - 119 °C. Nach Umkristallisieren aus Isoproanol/Wasser schmilzt das Produkt bei 124 °C. Ausbeute 61 %

b) In eine Lösung von 1,6 g NaOH in 30 ml Wasser gibt man 4,0 g 2-(4,4'-Difluorbiphenyl-2-yl)-3,4,4-trimethyl-4,5-dihydro-1,3-oxazolium-methylsulfat (Verb. VIII) und 30 ml Methanol. Nach 2 Stunden bei 65 °C wird die Reaktionslösung mit 2 N HCl angesäuert und i. Vak. eingedampft. Der Rückstand wird in Isopropanol /$H_2O$ 1:4 umkristallisiert und getrocknet.
Ausbeute: 2,25 g (99 %) mit Fp. 124 °C

c) In analoger Weise wird aus dem entsprechenden Jodid VIII (aus 4.a)) die Carbonsäure I nach Umkristallisation in einer Ausbeute von 92,8 % gewonnen.

d) Eintopfreaktion:

100 g (0,348 Mol) 2-(4,4'-Difluorbiphenyl-2-yl)-4,4-dimethyl-4,5-dihydro-1,3-oxazol (Verbindung VII) werden in 100 ml Aceton mit 40 ml (0,417 Mol) Dimethylsulfat 1,5 Stunden unter Rückfluß erwärmt. Während der Reaktion fällt ein farbloser Niederschlag aus. In die Suspension tropft man anschließend 342 ml (1,54 Mol) 18 %ige Natronlauge und destilliert gleichzeitg das Aceton ab. Bei einer Innentemperatur von etwa 90 °C wird anschließend unter Rückfluß erhitzt, bis die Verseifung beendet ist (etwa 1 Stunde). Man kühlt auf 20 °C, wobei sich das Reaktionsgemisch in zwei Phasen trennt. Die untere Phase wird verworfen, die obere zweimal mit je 100 ml Toluol gewaschen und dann mit Aktivkohle geklärt. Unter Kühlung wird mit konzentrierter Salzsäure angesäuert, wobei die kristalline, farblose 4,4'-Difluorbiphenyl-2-carbonsäure ausfällt. Es wird abgesaugt, mit Wasser gewaschen und i. Vak. bei 30 °C getrocknet.
Ausbeute 70,1 g (86,0 %)

Die nach Beispiel I erhaltene Carbonsäure kann wie folgt zu 2,7-Difluor-9-fluorenon (IX) cyclisiert werden:

2,34 g 4,4'-Difluorbiphenyl-2-carbonsäure werden bei 40 -50 °C zu 35 g Polyphosphorsäure gegeben. Unter Rühren erwärmt man 1 Stunde auf 100 °C. Die gelbe Suspension gießt man danach auf 150 ml Eiswasser. Der gelbe Niederschlag wird abgesaugt und gründlich mit Wasser gewaschen.
Ausbeute: 2,11 g (98 %) Verbindung IX
Fp. 208 - 210 °C

**Ansprüche**

1. Biphenylcarbonsäuren der Formel I

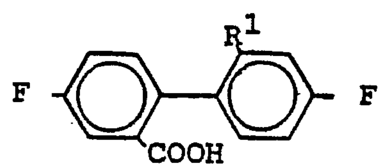

I

worin $R^1$ Wasserstoff oder Methoxy bedeutet.

2. Verfahren zur Herstellung von Biphenylcarbonsäuren der Formel I, dadurch gekennzeichnet, daß man a) eine 2-Methoxybenzoesäure der Formel II

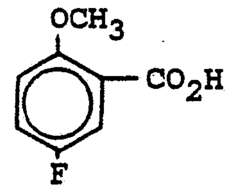

II

oder ein reaktives Derivat dieser Säure mit einem Ethanolamin der Formel III

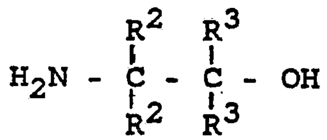

III

worin $R^2$ und $R^3$ Wasserstoff oder $(C_1-C_3)$-Alkyl, jedoch $R^2$ und $R^3$ nicht gleichzeitig Alkyl bedeuten, zu einem Amid der allgemeinen Formel IV

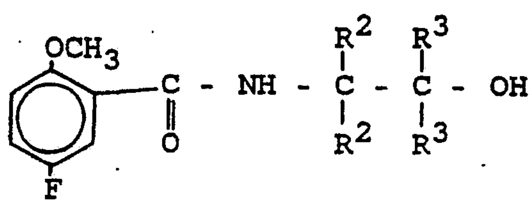

IV

worin $R^2$ und $R^3$ die zur Formel III angegebenen Bedeutungen haben, umsetzt, das Amid der Formel IV anschließend zu einem Dihydrooxazol der Formel V

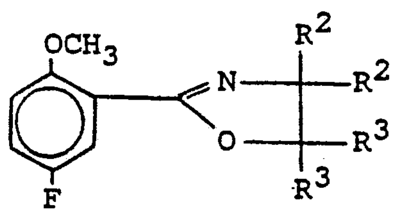

V

worin $R^2$ und $R^3$ die zur Formel III angegebenen Bedeutungen haben, cyclisiert, das Dihydrooxazol der Formel V anschließend mit einem metallorganischen Reagens der Formel VI

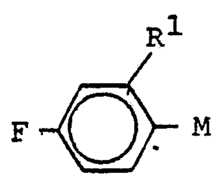

VI

worin $R^1$ die zur Formel I angegebene Bedeutung hat und M Lithium, Zn-Hal oder Mg-Hal und Hal Chlor, Brom oder Jod bedeuten, zu einem Biphenylderivat der allgemeinen Formel VII

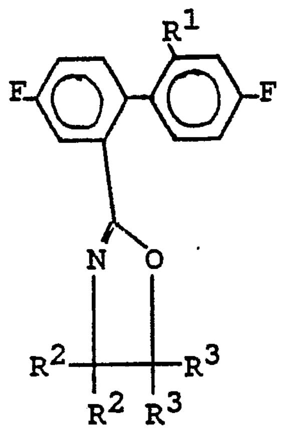

VII

worin $R^1$ die zur Formel I und $R^2$ und $R^3$ die zur Formel III angegebenen Bedeutungen haben, umsetzt, das erhaltene Biphenylderivat der Formel VII gegebenenfalls nach Überführung in die entsprechende alkylierte Verbindung der Formel VIII

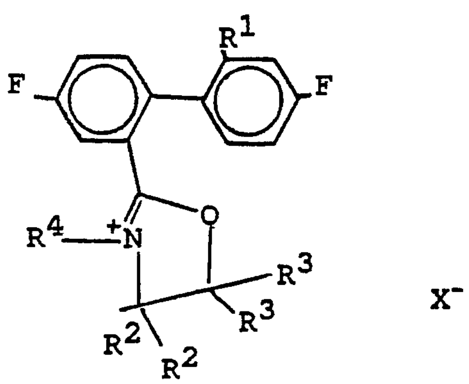

VIII

worin $R^1$ die zur Formel I und $R^2$ und $R^3$ die zur Formel III angegebenen Bedeutungen haben, $R^4$ ($C_1$-$C_3$)-Akyl bedeutet und $X^-$ Chlorid, Bromid, Jodid, Methylsulfat oder Ethylsulfat bedeutet, zur Carbonsäure der Formel I

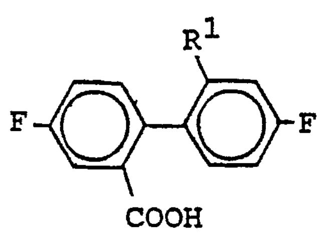

I

worin $R^1$ die zur Formel I angegebene Bedeutung hat hydrolysiert.

3. Verbindungen der Formel V.

4. Verbindungen der Formeln VII und VIII.

Patentansprüche für folgenden Vertragsstaat : ES.

1. Verfahren zur Herstellung von Biphenylcarbonsäuren der Formel I

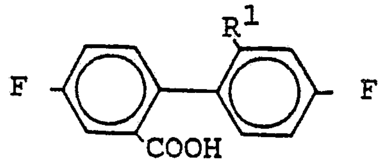

I

worin $R^1$ wasserstoff oder Methoxy bedeutet, dadurch gekennzeichnet, daß man
a) eine 2-Methoxybenzoesäure der Formel II

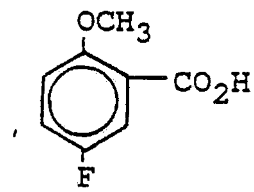

II

oder ein reaktives Derivat dieser Säure mit einem Ethanolamin der Formel III

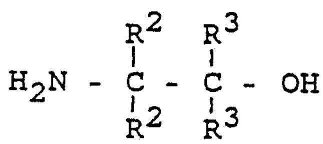

III

worin $R^2$ und $R^3$ Wasserstoff oder ($C_1$-$C_3$)-Alkyl, jedoch $R^2$ und $R^3$ nicht gleichzeitig Alkyl bedeuten, zu einem Amid der allgemeinen Formel IV

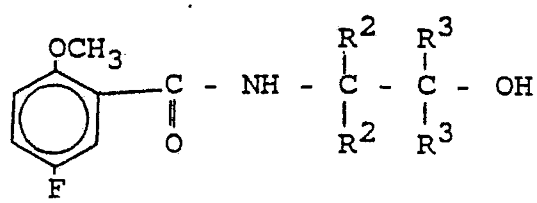

IV

worin $R^2$ und $R^3$ die zur Formel III angegebenen Bedeutungen haben, umsetzt, das Amid der Formel IV anschließend zu einem Dihydrooxazol der Formel V

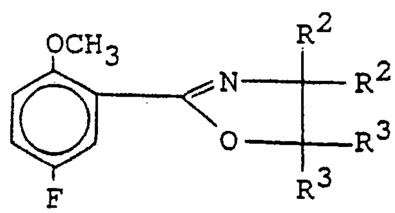

V

worin $R^2$ und $R^3$ die zur Formel III angegebenen Bedeutungen haben, cyclisiert, das Dihydrooxazol der Formel V anschließend mit einem metallorganischen Reagens der Formel VI

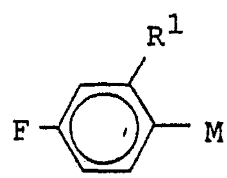

VI

worin $R^1$ die zur Formel I angegebene Bedeutung hat und M Lithium, Zn-Hal oder Mg-Hal und Hal Chlor, Brom oder Jod bedeuten, zu einem Biphenylderivat der allgemeinen Formel VII

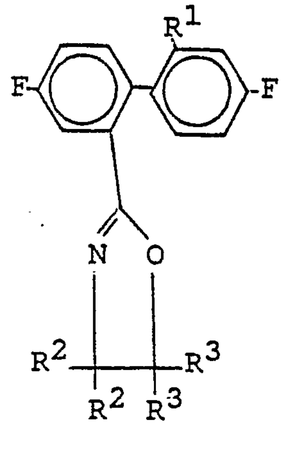

VII

worin $R^1$ die zur Formel I und $R^2$ und $R^3$ die zur Formel III angegebenen Bedeutungen haben, umsetzt, das erhaltene Biphenylderivat der Formel VII gegebenenfalls nach Überführung in die entsprechende alkylierte Verbindung der Formel VIII

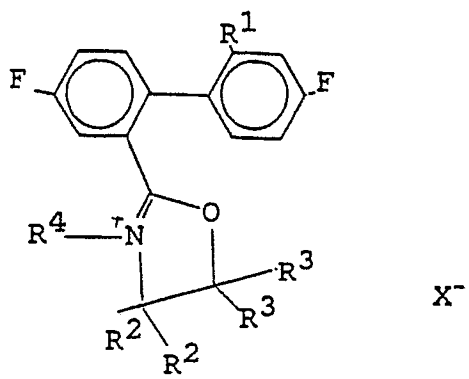

VIII

worin $R^1$ die zur Formel I und $R^2$ und $R^3$ die zur Formel III angegebenen Bedeutungen haben, $R^4$ $(C_1\text{-}C_3)$-Akyl bedeutet und $X^-$ Chlorid, Bromid, Jodid, Methylsulfat oder Ethylsulfat bedeutet, zur Carbonsäure der Formel I

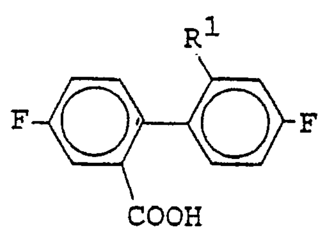

I

worin $R^1$ die zur Formel I angegebene Bedeutung hat hydrolysiert.

2. Verfahren gemäß Anspruch 1, dadurch gekennzeichnet, daß man eine Verbindung der Formel V isoliert.

3. Verfahren gemäß Anspruch 1, dadurch gekennzeichnet, daß man eine Verbindung der Formel VII oder VIII isoliert.